# EUROPÄISCHE PATENTANMELDUNG

(11) **EP 4 302 786 A1**
(43) Veröffentlichungstag der Anmeldung: **10.01.2024**
(21) Anmeldenummer: 22182833.8
(22) Anmeldetag: 04.07.2022
(51) Int. Cl.: A61L 2/00, A61L 2/10, A61L 2/24

(54) **STRAHLUNGSVORRICHTUNG ZUR DESINFEKTION VON ZAHNTECHNIK UND VERFAHREN ZU DEREN ÜBERWACHUNG**

(71) Anmelder: Zubler Gerätebau GmbH, 89081 Ulm (DE)
(72) Erfinder: ZUBLER, Kurt, 89233 Neu-Ulm (DE); STOCK, Karl, 73479 Ellwangen (DE); STEGMAYER, Thomas, 89511 Köningsbronn (DE); DOLP, Frank, 87773 Pleß a.d. Iller (DE); WITTIG, Rainer, 89081 Ulm (DE)
(74) Vertreter: Stolmár & Partner Patentanwälte PartG mbB

(57) **Zusammenfassung**

Offenbart ist eine Strahlungsvorrichtung (100) zur Desinfektion von Zahntechnik, umfassend eine Strahlungskammer (150) mit zumindest einer Öffnung (159) zum Be- und Entladen der Strahlungskammer (150) und einen mittig in der Strahlungskammer (150) angeordneten Stellplatz (160) für die zu desinfizierende Zahntechnik, eine Tür (140) zum Verschließen der Öffnung (159) der Strahlungskammer (150), und zumindest eine Strahlungsquelle, insbesondere zumindest zwei Strahlungsquellen mit jeweils einer Vielzahl von UVC-LED, die an zumindest zwei unterschiedlichen, insbesondere gegenüberliegenden Seiten (151) der Strahlungskammer (150) angeordnet sind, dadurch gekennzeichnet, dass die Strahlungsvorrichtung (100) vier oder mehr Sensoren (195) zur Strahlungserfassung aufweist, die zur Messung der Strahlung innerhalb der Strahlungskammer (150) vorgesehen sind, wobei in unterschiedlichen Kantenbereichen der Strahlungskammer (150) jeweils zumindest ein Sensor (195) vorgesehen ist.

## Beschreibung

### Gebiet der Erfindung

Die Erfindung betrifft eine Strahlungsvorrichtung zur Desinfektion von Zahntechnik und ein Verfahren zu deren Überwachung.

### Stand der Technik

Im Stand der Technik sind verschiedene Desinfektionslösungen für Zahntechnik bekannt. Diese sind beispielsweise das Tauch-, Sprüh- und Wischdesinfektionsverfahren. Bei diesen Desinfektionsverfahren kommen als Desinfektionsmittel chemische Wirkstoffe zum Einsatz, beispielsweise Aldehyde, quartäre Ammoniumverbindungen, Alkohole oder Alkylamine, die insbesondere bakterizid, tuberkulozid, levurozid und viruzid wirken.

Bei der Tauchdesinfektion wird die zu desinfizierende Zahntechnik, hierbei insbesondere Handwerkzeuge und Abdrücke, in einem Behältnis abgelegt, in dem Desinfektionsmittel enthalten ist. Anschließend wirkt das Desinfektionsmittel über einen vorgegebenen Zeitraum auf die Zahntechnik ein, teilweise bis zu 60 Minuten lang. Dabei werden je nach Fassungsvermögen des Behältnisses beispielsweise mehrere Liter Desinfektionsmittel benötigt, das je nach Verschmutzungsgrad der Zahntechnik teilweise bereits nach einem Desinfektionsgang ausgewechselt werden muss. Nach dem Einwirken wird die Zahntechnik aus dem Behältnis herausgenommen und muss getrocknet werden. Dieses Desinfektionsverfahren benötigt dementsprechend viel Zeit, händig auszuführende Zusatzschritte und Verbrauchsmaterialien. Außerdem ist die Tauchdesinfektion für Gipsmodelle oder andere Modelle aus Materialien, die Flüssigkeit absorbieren, nicht geeignet.

Bei der Sprühdesinfektion wird die zu desinfizierende Zahntechnik, hierbei insbesondere Abdrücke, in einem Behältnis abgelegt und anschließend mit Desinfektionsmittel besprüht. Das Desinfektionsmittel wird dabei druckluftunterstützt als Aerosol auf die Zahntechnik aufgetragen. Nach einer Einwirkzeit von beispielsweise 2 Minuten wird die Zahntechnik mit Wasser gespült. Anschließend muss die Zahntechnik getrocknet werden. Gegenüber der Tauchdesinfektion ergibt sich hier ein wesentlich schnelleres Verfahren, allerdings werden zusätzlich ein Wasseranschluss und ein Druckluftanschluss mit entsprechender Versorgung benötigt, wodurch die Prozesskosten steigen.

Bei der Wischdesinfektion wird die zu desinfizierende Zahntechnik mit Desinfektionstüchern abgewischt. Dieses Desinfektionsverfahren wird ausschließlich händig ausgeführt und ist wesentlich weniger gründlich als die Tauchdesinfektion und die Sprühdesinfektion.

Ferner sind Desinfektionsvorrichtungen bekannt, bei denen mittels Strahlung, beispielsweise mittels UVC-Strahlung Oberflächen oder kleine Gegenstände desinfiziert werden. Dabei kann aber kein Rückschluss darauf gezogen werden, welche Stellen der zu desinfizierenden Strukturen bereits ausreichend desinfiziert sind, wodurch sicherheitshalber in einem wesentlich größeren Umfang bestrahlt wird, als tatsächlich benötigt würde. Dadurch weisen strahlungsbasierte Desinfektionsverfahren eine geringe Energie- und Zeiteffizienz und teilweise auch eine geringe Gründlichkeit auf, insbesondere an Stellen, an denen die Strahlung nicht eingewirkt hat.

Aus dem Stand der Technik bekannte Desinfektionsverfahren für Zahntechnik sind daher entweder kostenintensiv, zeitintensiv, zu wenig gründlich oder benötigen eine Vielzahl händig auszuführender Schritte.

### Darstellung der Erfindung

Aufgabe der vorliegenden Erfindung ist es daher, eine Desinfektion von Zahntechnik zu ermöglichen, die gründlich - das heißt zu einer guten Desinfektionsqualität führt -, zügig und kostengünstig - das heißt beispielsweise energie- und zeiteffizient - ist sowie möglichst wenig händige Schritte bedarf.

Die Aufgabe wird gelöst durch eine Strahlungsvorrichtung nach Anspruch 1, eine Strahlungsvorrichtung nach Anspruch 0 sowie ein Verfahren nach Anspruch 0.

Weitere die Erfindung ausgestaltende Merkmale sind in den abhängigen Ansprüchen enthalten.

Eine erfindungsgemäße Strahlungsvorrichtung zur Desinfektion von Zahntechnik umfasst eine Strahlungskammer mit zumindest einer Öffnung zum Be- und Entladen der Strahlungskammer und einen insbesondere mittig in der Strahlungskammer angeordneten Stellplatz für die zu desinfizierende Zahntechnik, eine Tür zum Verschließen der Öffnung der Strahlungskammer, und zumindest eine Strahlungsquelle, insbesondere zumindest zwei Strahlungsquellen mit jeweils einer Vielzahl von UVC-LED, die an zumindest zwei unterschiedlichen, insbesondere gegenüberliegenden Seiten der Strahlungskammer angeordnet sind, dadurch gekennzeichnet, dass die Strahlungsvorrichtung vier oder mehr Sensoren zur Strahlungserfassung aufweist, die zur Messung der Strahlung innerhalb der Strahlungskammer vorgesehen sind, wobei in unterschiedlichen Kantenbereichen der Strahlungskammer jeweils zumindest ein Sensor vorgesehen ist.

Als Zahntechnik werden erfindungsgemäß insbesondere Werkzeuge, Implantate, Prothesen, Aufsätze, Modelle, Abdrücke, Abformlöffel, Artikulatoren und dergleichen verstanden, die in und für die zahntechnische und kieferorthopädische Behandlung oder auch beim Austausch zwischen Zahnarzt und Dentallabor Anwendung finden. Prinzipiell sind die erfindungsgemäßen Strahlungsvorrichtungen auch zur Desinfektion von weiterer Medizintechnik geeignet.

Als Strahlung wird erfindungsgemäß elektromagnetische Strahlung verstanden, vorzugsweise UV-Strahlung, besonders vorzugsweise UV-C-Strahlung, die eine Wellenlänge im Bereich von 200-320 nm, vorzugsweise von 230-290 nm oder weiter vorzugsweise von 250-280 nm, insbesondere 254-268 nm oder genau die Randwerte 254 nm oder 268 nm aufweist.

Als Kantenbereich wird erfindungsgemäß der Bereich der Strahlungskammer verstanden, an beziehungsweise in dem unterschiedliche Seiten beziehungsweise Wände, der Boden, die Tür oder die Decke aufeinander treffen. Vorzugsweise ist die Strahlungskammer im Wesentlichen quaderförmig ausgebildet.

In den Kantenbereichen ist der Anteil an direkter Strahlung einzelner LEDs besonders niedrig und man erhält ein Mischsignal, im Idealfall aus Strahlanteilen aller LEDs. Somit kann durch die Anordnung der Sensoren in den Kantenbereichen der Strahlungskammer, insbesondere in den am Boden befindlichen Kantenbereichen, die Strahlungsverteilung in der Kammer besser erfasst und dadurch der Strahlungsverlust genauer gemessen werden. Dadurch können sowohl Rückschlüsse auf die von der Zahntechnik absorbierte Strahlung gezogen werden als auch, insbesondere bei leerer Kammer, auf den Strahlverlust durch Leistungsabfall oder Ausfall einer oder mehrerer LEDs oder durch Verschmutzung der Wände. Anhand der zeitlichen Verläufe können ferner auch Rückschlüsse auf den Desinfektionszustand der Zahntechnik gezogen werden. Derart wird es ermöglicht, zeit- und energieeffizient zu desinfizieren oder auch verschiedene Zeitfenster festgestellt werden, in denen bestimmte Zahntechnik zuverlässig desinfiziert werden kann. Insbesondere können gegebenenfalls die Strahlungsquellen je nach Bedarf mit veränderter Leistung betrieben oder die Desinfektion beendet werden, wenn der sensorisch ermittelte Absorptionsgrad der Strahlung (die schon von der Zahntechnik absorbierte Strahlung) einen festgelegten Zielwert erreicht hat.

Vorzugsweise weist die Strahlungsvorrichtung zumindest zwei Strahlungsquellen auf, die an gegenüberliegenden Seiten der Strahlungskammer angeordnet sind, insbesondere an der Decke und am Boden und/oder an den gegenüberliegenden Wänden. Derart wird es ermöglicht, dass je nach gegebenenfalls sensorisch ermitteltem Desinfektionsbedarf der Zahntechnik die Leistung einer beziehungsweise beider Strahlungsquellen angepasst werden kann, wodurch eine höhere Zeit- und Energieeffizienz ermöglicht wird.

Vorzugsweise sind die Strahlungsquellen der gegenüberliegenden Seiten miteinander ausgerichtet. Das bedeutet, dass die Strahlungsquellen direkt gegenüberliegen und die einzelnen Sensoren so ausgerichtet sind, dass andere Sensoren den Strahlungsschatten durch ein Objekt erreichen und bestrahlen können. Insbesondere können die Strahlungsquellen bewegliche Elemente aufweisen, so dass die Strahlungsquellen tatsächlich insbesondere automatisiert in jeweils eine vorbestimmte Position gebracht werden können. Miteinander ausgerichtet umfasst aber auch eine Ausführungsform, in der die Strahlungsquellen direkt aufeinander gerichtet sind. Derart wird der mit Strahlung beaufschlagte Oberflächenanteil der zu desinfizierenden Zahntechnik maximiert, wodurch die Prozesseffizienz gesteigert werden kann.

Vorzugsweise sind die Sensoren 8 cm oder näher am Rand der jeweiligen Seite angeordnet, insbesondere 6 cm oder 5 cm, weiter bevorzugt am äußersten Rand. Als äußerster Rand wird erfindungsgemäß eine Anordnung direkt an der Seitenwand beziehungsweise der Decke beziehungsweise dem Boden beziehungsweise der Tür verstanden, an deren Enden keine radialen Übergänge vorgesehen sind beziehungsweise am Beginn des jeweiligen radialen Übergangs, falls ein solcher vorgesehen ist. Dadurch wird der messbare Anteil der in der Kammer vorhandenen Strahlung maximiert, wodurch wiederum mit höherer Genauigkeit auf den Anteil der von der Zahntechnik absorbierten Strahlung rückgeschlossen werden kann, und damit auf eine Desinfektions- und möglicherweise sogar Sterilisationswirkung der Strahlung. Insbesondere sind die Sensoren nicht gegenüber den Strahlungsquellen angeordnet, so dass die Strahlung der Strahlungsquellen nicht direkt auf die Sensoren trifft und möglicherweise das Messergebnis verfälscht. Weiter bevorzugt sind mehrere Sensoren an gegenüberliegenden Seiten vorgesehen und insbesondere gegenüberliegend angeordnet.

Eine alternative erfindungsgemäße Strahlungsvorrichtung zur Desinfektion von Zahntechnik umfasst eine Strahlungskammer mit zumindest einer Öffnung zum Be- und Entladen der Strahlungskammer und einen mittig in der Strahlungskammer angeordneten Stellplatz für die zu desinfizierende Zahntechnik, eine Tür zum Verschließen der Öffnung der Strahlungskammer, zumindest eine Strahlungsquelle, insbesondere zumindest zwei Strahlungsquellen mit jeweils einer Vielzahl von UVC-LED, die an zumindest zwei unterschiedlichen, insbesondere gegenüberliegenden Seiten der Strahlungskammer angeordnet sind, ein oder mehrere Sensoren zur Strahlungserfassung, die zur Messung der Strahlung innerhalb der Strahlungskammer angeordnet sind, dadurch gekennzeichnet, dass der Stellplatz als drehbare Plattform ausgebildet ist und die Strahlungsvorrichtung ferner einen Antrieb aufweist, der die drehbare Plattform während des Betriebs der Strahlungsvorrichtung antreibt.

Prinzipiell kann die Plattform dabei um eine beliebige Achse drehbar gelagert sein und in Bezug auf die Strahlungskammer mit einer beliebigen äußeren Begrenzung verbunden sein. Um die Objekte dann auf der Plattform zu halten können Befestigungselemente wie fest an der Plattform installierte Greifer, Schraubzwingen oder Gurte vorgesehen sein, oder die zu behandelnde Medizintechnik magnetisch auf der Plattform befestigt werden (wo möglich). Es ist jedoch bevorzugt, dass die Plattform mit dem Boden der Strahlungskammer verbunden ist und eine Drehachse aufweist, die senkrecht zum Boden der Strahlungskammer positioniert ist.

Durch die drehbare Ausführung der Plattform kann es ermöglicht werden, auch mit einer geringen Anzahl an Sensoren und Strahlungsquellen einen hohen Desinfektionsgrad zu erreichen, indem die Zahntechnik je nach oberflächlichem Desinfektionsbedarf in Bezug auf die Strahlungsquelle in deren effektivsten Wirkbereich beziehungsweise in deren effektivste Wirkrichtung ausgerichtet wird. Bei dauerhafter Rotation des Drehtellers gibt es dann keine Bereiche in der Strahlungskammer, die durch die zu desinfizierende Zahntechnik dauerhaft abgeschattet werden (da die Zahntechnik mit dem Drehteller rotiert) und auch die Zahntechnik selber kann durch die Rotation gleichmäßiger bestrahlt werden, da sich die Oberflächen dann in unterschiedlichen Positionen zu den Strahlungsquellen befinden. Derart kann eine energieeffiziente und günstige Strahlungsvorrichtung bereitgestellt werden.

Vorzugsweise weist die Strahlungsvorrichtung neben der drehbaren Plattform vier oder mehr Sensoren zur Strahlungserfassung auf, die zur Messung der Strahlung innerhalb der Strahlungskammer vorgesehen sind, wobei in unterschiedlichen Kantenbereichen der Strahlungskammer jeweils zumindest ein Sensor vorgesehen ist. Derart kann die Prozesseffizienz erhöht werden.

Vorzugsweise ist die die drehbare Plattform aus einem strahlungsdurchlässigen Material, insbesondere aus Quarzglas ausgebildet. Derart kann es ermöglicht werden, dass auch teilweise von der drehbaren Plattform verdeckte Bereiche der zu desinfizierenden Zahntechnik mit Strahlung beaufschlagt werden, wodurch die Desinfektionsqualität gesteigert werden kann. Dies betrifft insbesondere Bereiche, die zum Boden der Strahlungskammer hin gerichtet sind.

Vorzugsweise ist an den Seiten, dem Boden und der Decke jeweils eine Strahlungsquelle vorgesehen. Derart können mehrere gegebenenfalls unterschiedlich ausgerichtete Oberflächenbereiche der zu desinfizierenden Zahntechnik gleichzeitig mit Strahlung beaufschlagt werden, wodurch eine geringere Desinfektionszeit erreicht werden kann und eine bessere Desinfektionsqualität.

Vorzugsweise gehen Kantenbereiche der Strahlungskammer mit einem Radius ineinander über. Derart kann insbesondere in den Kantenbereichen die Reflektionsneigung der Strahlungskammer erhöht und deren Absorptionsneigung verringert werden, wodurch wiederum die Prozesseffizienz und die Desinfektionsqualität erhöht werden können.

Vorzugsweise umfasst die Strahlungsquelle eine Vielzahl von Strahlungselementen, die in regelmäßigen Abständen in Reihen und Spalten angeordnet sind, wobei die Strahlungselemente insbesondere als UVC-LED ausgebildet sind. Derart wird eine effektive Strahlungsemission ermöglicht, wodurch wiederum die Prozesseffizienz gesteigert werden kann.

Vorzugsweise sind die Strahlungsquellen und die einzelnen Strahlungselemente einzeln ansteuerbar. Derart kann je nach sensorisch ermitteltem, in Bezug auf die Zahntechnik räumlich gegebenenfalls unterschiedlichem Desinfektionsbedarf die Strahlungsleistung angepasst werden, wodurch die Energie- und die Prozesseffizienz gesteigert werden können.

Vorzugsweise umfasst die Strahlungsvorrichtung ferner eine Anzeige, die insbesondere als Touch-Display ausgebildet ist, wobei mittels der Anzeige die Strahlungsvorrichtung steuerbar ist. Die Anzeige kann an der Außenseite der Strahlungsvorrichtung vorgesehen sein, wodurch eine Bedienbarkeit unmittelbar an der Strahlungsvorrichtung ermöglicht wird. Die Anzeige kann aber auch entfernt von der Strahlungsvorrichtung vorgesehen sein, wodurch eine Bedienbarkeit gegebenenfalls mehrerer Strahlungsvorrichtung aus einem zentralen Kontrollraum heraus ermöglicht wird. Derart wird eine einfache Steuerung der Strahlungsvorrichtung ermöglicht, die durch Vorsehen eines Touch-Displays zusätzlich vereinfacht wird, wodurch die Bedienbarkeit verbessert wird.

Vorzugsweise weist eine innere Oberfläche der Strahlungskammer zumindest teilweise, vorzugsweise im Wesentlichen vollständig, eine freie Oberflächenenergie von maximal 40 mN/m, vorzugsweise 30 mN/m, besonders bevorzugt 25 mN/m auf. Die vorgesehene freie Oberflächenenergie ist dadurch lokal verringert - im Vergleich beispielsweise zu einer metallisch ausgeführten Strahlungskammer, wie einer Strahlungskammer aus Edelstahl, wodurch lokal das Anhaften von Partikeln, die während der Desinfektion abgetragen werden, sollen beziehungsweise sterilisiert werden sollen, an der Innenseite der Strahlungskammer verringert wird. Derart wird die Desinfektionsqualität verbessert. Diese vorhergehend genannte Innere Oberfläche der Strahlungskammer kann in einer alternativen Ausführungsform auch in einer Strahlungsvorrichtung zur Desinfektion von Zahntechnik vorgesehen sein, die eine Strahlungskammer umfasst mit zumindest einer Öffnung zum Be- und Entladen der Strahlungskammer und einen in der Strahlungskammer angeordneten Stellplatz für die zu desinfizierende Zahntechnik, eine Tür zum Verschließen der Öffnung der Strahlungskammer, und zumindest eine Strahlungsquelle, insbesondere zumindest zwei Strahlungsquellen mit jeweils einer Vielzahl von UVC-LED, die an zumindest zwei unterschiedlichen, insbesondere gegenüberliegenden Seiten der Strahlungskammer angeordnet sind,

Die lokale Verringerung der freien Oberflächenenergie kann durch Beschichtung der Innenseite der Strahlungskammer beziehungsweise der Tür mit entsprechendem Material, beispielsweise mit einer entsprechenden Folie erreicht werden oder mit einem lokal entsprechend andersartig vorgesehenen Material. Die entsprechende innere Oberfläche kann dabei Seiten beziehungsweise Wände und/oder die Decke und/oder den Boden der Strahlungskammer und/oder auch die Tür betreffen.

Vorzugsweise weist die innere Oberfläche der Strahlungskammer zumindest teilweise, vorzugsweise im Wesentlichen vollständig einen Reflexionsgrad von mindestens 80 %, vorzugsweise 90 %, besonders bevorzugt 93 % auf. Vorzugsweise weist die innere Oberfläche dabei eine möglichst diffus in alle Raumrichtungen reflektierende Oberfläche auf, wodurch eine gewünschte möglichst homogene Orts- und Winkelverteilung der Strahlung in der gesamten Kamer erzielt wird. Derart wird der Anteil an Strahlung erhöht, der - da er nicht absorbiert wird - tatsächlich eine Desinfektionswirkung entfaltet, wodurch wiederum die Prozess- und Energieeffizienz erhöht werden kann.

Vorzugsweise ist die innere Oberfläche der Strahlungskammer zumindest teilweise, vorzugsweise im Wesentlichen vollständig aus Polytetrafluorethylen, vorzugsweise aus gesintertem Polytetrafluorethylen ausgebildet. Derart wird eine hohe Temperatur-, Strahlungs- und Abrasionsbeständigkeit der inneren Oberfläche der Strahlungskammer erreicht, wodurch die Standzeit der Strahlungsvorrichtung erhöht werden kann und Wartungskosten minimiert werden können.

In einer bevorzugten Ausführungsform der Erfindung weist eine Strahlungsvorrichtung die Sensoren im Kantenbereich und/oder den Drehteller und/oder die Beschichtung auf der Innenseite der Strahlungskammer auf. Das gilt auch für die spezielleren Merkmale der jeweiligen Ausgestaltungen.

Ein erfindungsgemäßes Verfahren zum Überwachen einer Strahlungsvorrichtung zum Desinfizieren von Zahntechnik, insbesondere eine wie vorstehend dargestellte Strahlungsvorrichtung, umfasst die Schritte des Erfassens der Parameter der zu desinfizierenden Zahntechnik, Startens des Betriebs der Strahlungsvorrichtung, kontinuierlichen Erfassens der Strahlungsdichte in der Strahlungskammer, Auswertens der erfassten Strahlungsdichte in Abhängigkeit der erfassten Zahntechnikparamter, und Ausgebens einer Fehlermeldung bei einer unerwarteten Abweichung der erfassten Strahlungsdichte während der Auswertung.

Vorzugsweise wird das Auswerten der erfassten Strahlungsdichte kontinuierlich während des Betriebs der Strahlungsvorrichtung ausgeführt. Derart kann unmittelbar auf das Erreichen eines Zielwerts hinsichtlich des Desinfektionsgrades reagiert werden, wodurch die Prozess- und Energieeffizienz gesteigert werden können.

### Kurze Beschreibung der Figuren

Fig. 1a zeigt eine Strahlungsvorrichtung mit geöffneter Tür in einer isometrischen Ansicht.
Fig. 1b zeigt die in Fig. 1a dargestellte Strahlungsvorrichtung mit geschlossener Tür in einer isometrischen Ansicht.
Fig. 2 zeigt die in den Fig. 1a und 1b dargestellte Strahlungsvorrichtung mit geöffneter Tür und ohne Gehäuse in einer isometrischen Ansicht.
Fig. 3 zeigt die in Fig. 2 dargestellte Strahlungsvorrichtungen ohne Tür in einer Frontalansicht.
Fig. 4a zeigt die in Fig. 3a dargestellte Strahlungsvorrichtung in einer isometrischen Ansicht von schräg oben.
Fig. 4b zeigt die die in Fig. 4a dargestellte Strahlungsvorrichtung in einer isometrischen Ansicht von schräg unten.
Fig. 5a zeigt eine Strahlungskammer in einer isometrischen Ansicht.
Fig. 5b zeigt ein Detail der in Fig. 5a dargestellten Strahlungskammer.

### Beschreibung der bevorzugten Ausführunasformen

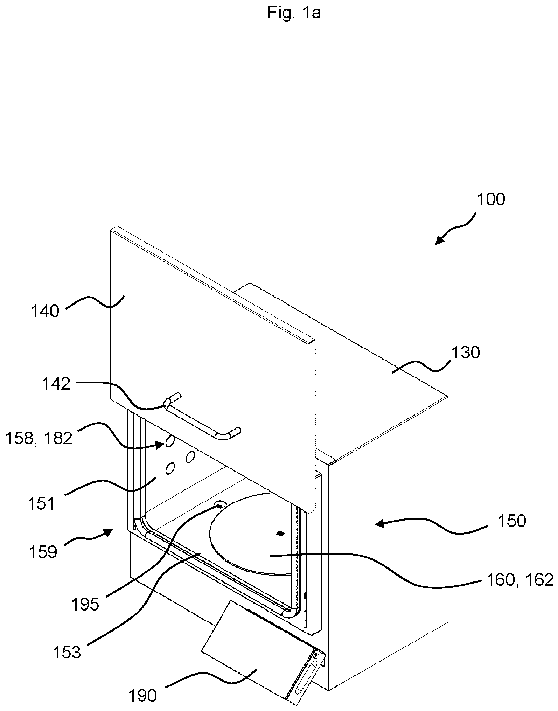

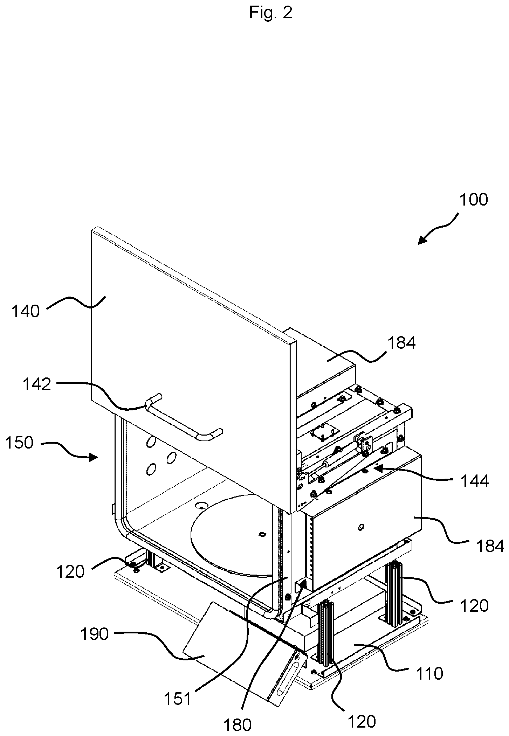

Fig. 1a zeigt eine Strahlungsvorrichtung 100 mit geöffneter Tür 140 in einer isometrischen Ansicht. Die Tür 140 ist nach oben hin geöffnet. Die Tür 140 weist einen Griff 142 auf, der außen an diese angebracht ist und mit dem diese händig geöffnet werden kann. Die Tür kann ferner zum automatisierten oder zum teilautomatisierten Öffnen ausgebildet sein. Die Strahlungsvorrichtung 100 weist eine Strahlungskammer 150 auf. Die Strahlungskammer 150 ist über eine Öffnung 159 mit zu desinfizierender Zahntechnik beladbar. Die Strahlungsvorrichtung 100 weist ferner ein Gehäuse 130 auf, mit dem die Strahlungskammer 150 eingehaust ist. Auf der Außenseite und unterhalb der Strahlungskammer 150 weist die Strahlungsvorrichtung 100 ferner eine Anzeige 190 auf. Auf dem Boden 153 der Strahlungskammer 150 ist ein Stellplatz 160 angeordnet, der hier als drehbare Plattform 162 ausgebildet ist. Neben der drehbaren Plattform 162 - in der Darstellung links - ist ein Sensor 195 angeordnet. Die Seite 151 der Strahlungskammer 150 weist eine Vielzahl an Aussparungen 158 auf, hinter denen jeweils eine Vielzahl von Strahlungselementen 182 positioniert ist - in der Darstellung wird lediglich exemplarisch auf eine Aussparung 158 und ein Strahlungselement 182 verwiesen. Mehrere Strahlungselemente 182 sind in einer Strahlungsquelle 180 angeordnet (hier nicht dargestellt, vergleiche beispielsweise Fig. 2). Die Anzeige 190 dient zum Steuern der Strahlungsvorrichtung 100 und zum Anzeigen von prozessrelevanten Informationen, wie Strahlungsparametern und dem Prozessstatus.

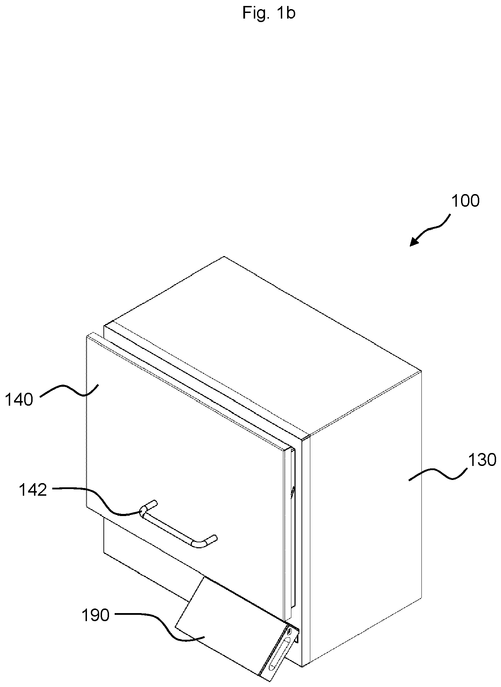

Fig. 1b zeigt die in Fig. 1a dargestellte Strahlungsvorrichtung 100 mit geschlossener Tür 140 in einer isometrischen Ansicht. Im geschlossenen Zustand verdeckt die Tür 140 die Strahlungskammer 150 vollständig (vergleiche Fig. 1a).

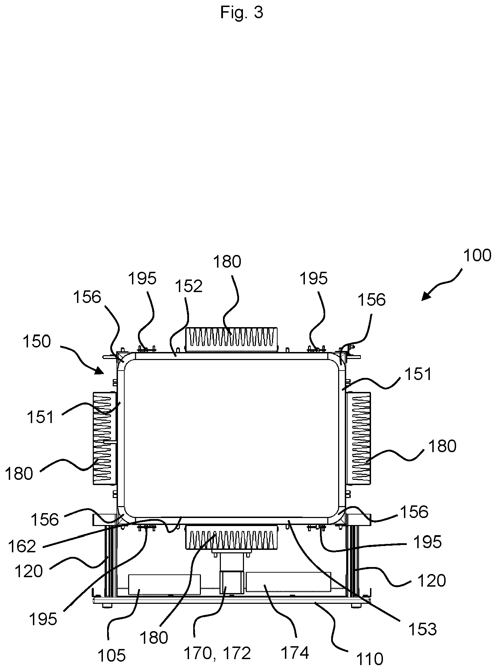

Fig. 2 zeigt die in den Fig. 1a und 1b dargestellte Strahlungsvorrichtung 100 mit geöffneter Tür 140 und ohne Gehäuse 130 (vergleiche Fig. 1a und 1b) in einer isometrischen Ansicht. Am oberen Ende der Strahlungskammer 150 befindet sich ein Klappmechanismus 144, der mit der Tür 140 verbunden ist und deren Öffnung beziehungsweise Schließung ermöglicht. Dieser Klappmechanismus 144 kann ferner beispielsweise als Schwenk- oder Schiebemechanismus ausgebildet sein und pneumatisch oder hydraulisch unterstützt sein. Die Strahlungskammer 150 ist über mehrere Standbeine 120 mit einer Basisplatte 110 verbunden beziehungsweise auf dieser abgestützt beziehungsweise fixiert. An der in der Fig. rechts dargestellten Seite 151 der Strahlungskammer 150 ist nach außen hin gerichtet eine Strahlungsquelle 180 angeordnet, die noch weiter nach außen hin von einer Abdeckung 184 abgedeckt ist. Die Abdeckung 184 überdeckt dabei die Längsfläche der Strahlungsquelle 180 vollständig, während an den Querflächen (hier ist nur eine Querfläche ersichtlich) der Strahlungsquelle 180 keine Überdeckung durch die Abdeckung 184 stattfindet. Neben der Vielzahl von Strahlungselementen 182 weist die Strahlungsquelle 180 ferner eine Trägerplatine, einen Kühlkörper - dessen Rippen sind beispielsweise in Fig. 3 ersichtlich - und bspw. einen Lüfter auf - um eine entsprechend kühlende Luftströmung zu ermöglichen sind die Querflächen wie oben beschrieben nicht überdeckt. In einer anderen Ausführungsform gibt es einen zentralen Lüfter hinter der Strahlungsvorrichtung für alle Strahlungsquellen. Dieser Lüfter bläst die Luft vorzugsweise weg von der Strahlungsvorrichtung und erzeugt so einen Unterdruck oder Sog, mit dem die Luft vor der Strahlungsvorrichtung zum Lüfter gesaugt wird. Die Abdeckungen dienen dann als Luftführungsbleche, die die angesaugte Luft zum Lüfter leiten.

Fig. 3 zeigt die in Fig. 2 dargestellte Strahlungsvorrichtung 100 ohne Tür 140 (vergleiche beispielsweise Fig. 2) in einer Frontalansicht. Die Strahlungskammer 150 weist jeweils an ihrer Decke 152, ihren Seiten 151 (hier zwei Seiten 151, jeweils eine Seite 151 links und eine Seite 151 rechts dargestellt) und ihrem Boden 153 eine Strahlungsquelle 180 auf. Unterhalb der unterhalb des Bodens 153 angeordneten Strahlungsquelle 180 ist ein als Motor 172 ausgebildeter Antrieb 170 angeordnet, der wiederum mit der innerhalb der Strahlungskammer 150 angeordneten drehbaren Plattform 162 verbunden ist. Der Motor 172 ist dabei insbesondere ein Schrittmotor und dazu eingerichtet, die drehbare Plattform 162 zu rotieren, sodass auf der drehbaren Plattform 162 angeordnete Zahntechnik ebenfalls rotiert wird. Derart können alle Bereiche der zu desinfizierenden Zahntechnik in die Strahlung gerichtet werden. Der Motor 172 ist ferner mit einer Motorsteuerung 174 verbunden, die dazu eingerichtet ist, den Motor 172 zu steuern. An der Decke 152 und an dem Boden 153 sind jeweils zwei oder mehrere Sensoren 195 angeordnet, mit denen von den Strahlungselementen 180 emittierte Strahlung messbar ist. Die Strahlungskammer 150 weist ferner vier Radien 156 auf, wobei die Radien 156 einen geschwungenen Übergang jeweils von der Decke 152 zu den Seiten 151 und von den Seiten 151 zu dem Boden 153 ausbilden. Derart wird die Reflektionsneigung der inneren Strahlungskammer 150 insbesondere in deren Kantenbereichen verstärkt, da die Übergänge zwischen den Seiten 151 zur Decke 152 beziehungsweise zum Boden 153 dann einen größeren Einfallswinkel hinsichtlich der eintreffenden Strahlung aufweisen und demzufolge in geringerem Maße als Strahlungsfänger beziehungsweise -absorber fungieren. Die Sensoren sind jeweils in der Nähe der Seiten 151 - ein Sensor pro Seite 151 und Boden 153 beziehungsweise pro Seite 151 und Decke 152 - und unmittelbar vor den Radien 156 angeordnet. Derart wird die am Sensor eintreffende und damit auch zu messende Strahlung maximiert, wodurch wiederum mit höherer Genauigkeit auf den Anteil der von der Zahntechnik absorbierten und damit eine Sterilisationswirkung entfaltenden Strahlung rückgeschlossen werden kann. Dadurch können die Strahlungsquellen 180 effizienter gesteuert werden und gleichzeitig kann die Desinfektionsqualität gesteigert werden. Unterhalb der Strahlungskammer 150 ist ferner eine Steuereinheit 105 angeordnet, mit der die Strahlungsquellen 180 und die Sensoren 195 steuerbar sind bzw. ausgelesen werden.

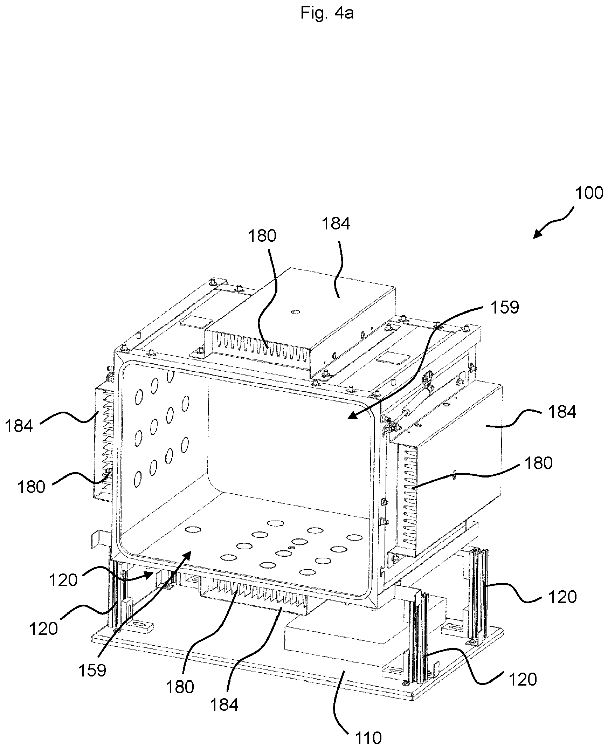

Fig. 4a zeigt die in Fig. 3a dargestellte Strahlungsvorrichtung 100 in einer isometrischen Ansicht von schräg oben. Jede der vier Strahlungsquellen 180 ist nach außen hin mit einer Abdeckung 184 überdeckt beziehungsweise abgedeckt. Wie vorhergehend erwähnt, dienen die Abdeckungen vorzugsweise auch zur Luftführung und damit zur Kühlung der Strahlungsquellen. Ferner ist hier ersichtlich, dass die Strahlungskammer 150 über vier Standbeine 120 auf der Basisplatte 110 abgestützt ist, wodurch eine hohe Stabilität erreicht werden kann. Durch das Anordnen und Ausrichten der Strahlungsquellen 180 in unterschiedliche Strahlungsrichtungen wird es insbesondere bei der vorzugsweisen Verwendung von UVC-LED als Strahlungselemente 182 ermöglicht, eine gleichmäßige Strahlstärke in der gesamten Strahlungskammer 150 zu erzeugen. Die in Fig. 4a dargestellte Strahlungskammer 150 weist zwei Öffnungen 159 auf, wobei die vordere im Betriebszustand durch die Tür 140 verschlossen ist und die hintere durch das Gehäuse 130. Die Innenseite der Tür 140 und die entsprechend die hintere Öffnung 159 verdeckende Innenseite des Gehäuses 130 werden auch als Teil der Strahlungskammer 150 verstanden und schließen diese nach vorne hin und nach hinten hin ab.

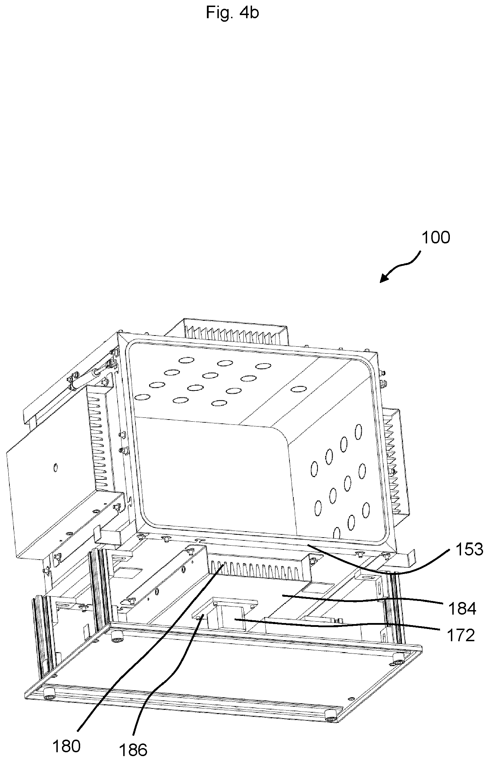

Fig. 4b zeigt die die in Fig. 4a dargestellte Strahlungsvorrichtung 100 in einer isometrischen Ansicht von schräg unten. Unterhalb der unterhalb des Bodens 153 angeordneten Strahlungsquelle 180 ist der Motor 172 über einen Flansch 186 mit der dazugehörigen Abdeckung 184 verbunden beziehungsweise über den Flansch 186 an dieser Abdeckung 184 befestigt.

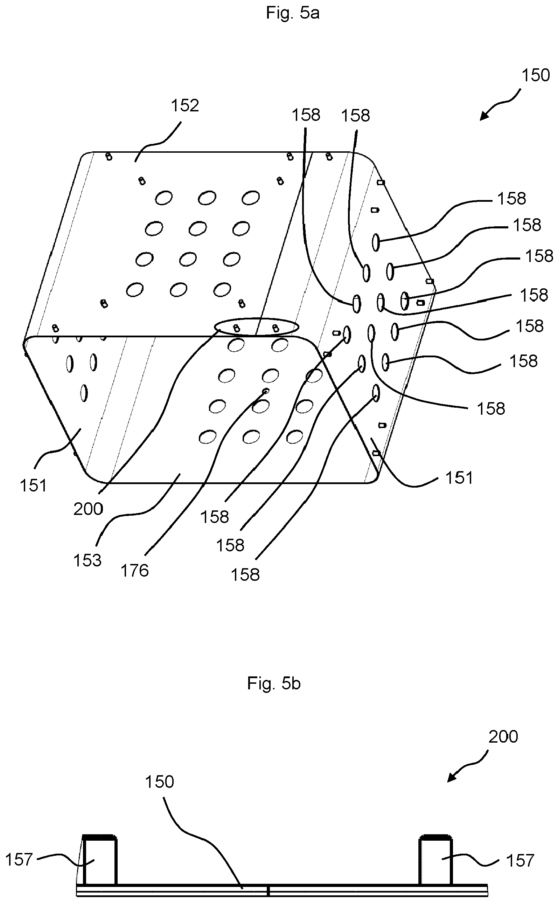

Fig. 5a zeigt eine Strahlungskammer 150 in einer isometrischen Ansicht. Die Strahlungskammer 150 weist jeweils an ihren Seiten 151, ihrem Boden 153 und ihrer Decke 152 zwölf Aussparungen 158 auf (hier werden lediglich exemplarisch für die rechte Seite 151 die entsprechenden Bezugszeichen dargestellt), wobei diese beziehungsweise die Strahlungsvorrichtung 100 derart ausgebildet sind, dass nach außen hin hinter den Aussparungen 158 jeweils ein Strahlungselement 182 positioniert ist (hier nicht dargestellt, vergleiche aber Fig. 1a). Die Aussparungen 158 beziehungsweise Strahlungselemente 182 sind dabei in regelmäßigen Abständen in Reihen und Spalten angeordnet. Derart wird aus vier Richtungen (zweimal von den Seiten 151 her, je einmal von der Decke 152 und dem Boden 153 her) ein gleichmäßiger Eintrag von Strahlung in die Strahlungskammer 150 hinein ermöglicht. Auf dem Boden 153 ist mittig zwischen den Aussparungen 158 ferner eine Motoraussparung 176 angeordnet, die eine Verbindung zwischen dem Motor 172 und der drehbaren Plattform 162 ermöglich (vergleiche Fig. 3 und 1a). In Fig. 5a sind keine Aussparungen für die Sensoren 195 dargestellt. Es versteht sich jedoch, dass die Strahlungskammer 150 auch entsprechende Aussparungen für die Sensoren 195 aufweist (vergleiche dazu beispielsweise Fig. 1a, 4a und 4b). Die Aussparungen 158 sind nach innen hin vorzugsweise mit strahlungsdurchlässigen Quarzglasronden abgedeckt, sodass sich in der Strahlungskammer 150 eine einfach zu reinigende und robuste Oberfläche ergibt. Gleichzeitig werden dadurch die hinter den Aussparungen 158 positionierten Strahlungselemente 182 geschützt. Das Gleiche kann für die Sensoren 195 vorgesehen sein - auch diese können durch entsprechende Quarzglasronden abgedeckt sein.

Fig. 5b zeigt ein Detail 200 der in Fig. 5a dargestellten Strahlungskammer 150. Das Detail 200 zeigt dabei zwei nach außen gerichtete Gewindebolzen 157, die an der Strahlungskammer 150 angeordnet sind und eine Befestigung beziehungsweise Verbindung der Strahlungskammer 150 mit dem Gehäuse 130 ermöglichen (vergleiche auch Fig. 1a und 2).

### Bezuaszeichenliste

- 100: Strahlungsvorrichtung
- 105: Steuereinheit
- 110: Basisplatte
- 120: Standbein
- 130: Gehäuse
- 140: Tür
- 142: Griff
- 144: Klappmechanismus
- 150: Strahlungskammer
- 151: Seite
- 152: Decke
- 153: Boden
- 155: Kantenbereich
- 156: Radius
- 157: Gewindebolzen
- 158: Aussparung
- 159: Öffnung
- 160: Stellplatz
- 162: drehbare Plattform
- 170: Antrieb
- 172: Motor
- 174: Motorsteuerung
- 176: Motoraussparung
- 180: Strahlungsquelle
- 182: Strahlungselement
- 184: Abdeckung
- 186: Flansch
- 190: Anzeige
- 195: Sensor
- 200: Detail

## Patentansprüche

1. Strahlungsvorrichtung (100) zur Desinfektion von Zahntechnik, umfassend eine Strahlungskammer (150) mit zumindest einer Öffnung (159) zum Be- und Entladen der Strahlungskammer (150) und einen in der Strahlungskammer (150) angeordneten Stellplatz (160) für die zu desinfizierende Zahntechnik,
eine Tür (140) zum Verschließen der Öffnung (159) der Strahlungskammer (150), und
zumindest eine Strahlungsquelle (180), insbesondere zumindest zwei Strahlungsquellen (180) mit jeweils einer Vielzahl von UVC-LED, die an zumindest zwei unterschiedlichen, insbesondere gegenüberliegenden Seiten (151) der Strahlungskammer (150) angeordnet sind,
**dadurch gekennzeichnet, dass**
die Strahlungsvorrichtung (100) vier oder mehr Sensoren (195) zur Strahlungserfassung aufweist, die zur Messung der Strahlung innerhalb der Strahlungskammer (150) vorgesehen sind, wobei in unterschiedlichen Kantenbereichen (155) der Strahlungskammer (150) jeweils zumindest ein Sensor (195) vorgesehen ist.

2. Strahlungsvorrichtung (100) nach Anspruch 1, bei der die Strahlungsvorrichtung (100) zumindest zwei Strahlungsquellen (180) aufweist, die an gegenüberliegenden Seiten (151) der Strahlungskammer (150) angeordnet sind, insbesondere an der Decke (152) und am Boden (153) oder an zwei gegenüberliegenden Wänden.

3. Strahlungsvorrichtung (100) nach Anspruch 2, bei der die Strahlungsquellen (180) der gegenüberliegenden Seiten (151) miteinander ausgerichtet sind.

4. Strahlungsvorrichtung (100) nach Anspruch 2 oder 3, bei der die Sensoren (195) 8 cm oder näher am Rand der jeweiligen Seite (151) angeordnet sind, insbesondere 6 cm oder 5 cm, weiter bevorzugt am äußersten Rand.

5. Strahlungsvorrichtung (100) zur Desinfektion von Zahntechnik, umfassend
eine Strahlungskammer (150) mit zumindest einer Öffnung (159) zum Be- und Entladen der Strahlungskammer (150) und einen mittig in der Strahlungskammer (150) angeordneten Stellplatz (160) für die zu desinfizierende Zahntechnik,
eine Tür (140) zum Verschließen der Öffnung (159) der Strahlungskammer (150), zumindest eine Strahlungsquelle (180), insbesondere zumindest zwei Strahlungsquellen (180) mit jeweils einer Vielzahl von UVC-LED, die an zumindest zwei unterschiedlichen, insbesondere gegenüberliegenden Seiten (151) der Strahlungskammer (150) angeordnet sind,
ein oder mehrere Sensoren (195) zur Strahlungserfassung, die zur Messung der Strahlung innerhalb der Strahlungskammer (150) angeordnet sind,
**dadurch gekennzeichnet, dass**
der Stellplatz (160) als drehbare Plattform (162) ausgebildet ist und die Strahlungsvorrichtung (100) ferner einen Antrieb (170) aufweist, der die drehbare Plattform (162) während des Betriebs der Strahlungsvorrichtung (100) antreibt.

6. Strahlungsvorrichtung (100) nach Anspruch 5 in Kombination mit zumindest einem der Ansprüche 1-4.

7. Strahlungsvorrichtung (100) nach Anspruch 5 oder 6, bei der die drehbare Plattform (162) aus einem strahlungsdurchlässigen Material, insbesondere aus Quarzglas ausgebildet ist.

8. Strahlungsvorrichtung (100) nach einem der vorhergehenden Ansprüche, bei der an den Seiten (151), dem Boden (153) und der Decke (152) jeweils eine Strahlungsquelle (180) vorgesehen ist.

9. Strahlungsvorrichtung (100) nach einem der vorhergehenden Ansprüche, bei der Kantenbereiche (155) der Strahlungskammer (150) mit einem Radius (156) ineinander übergehen.

10. Strahlungsvorrichtung (100) nach einem der vorhergehenden Ansprüche, bei der die Strahlungsquelle (180) eine Vielzahl von Strahlungselementen (182) umfasst, die in regelmäßigen Abständen in Reihen und Spalten angeordnet sind, wobei die Strahlungselemente (182) insbesondere als UVC-LED ausgebildet sind.

11. Strahlungsvorrichtung (100) nach Anspruch 10, bei der die Strahlungsquellen (180) und die einzelnen Strahlungselemente (182) einzeln ansteuerbar sind.

12. Strahlungsvorrichtung (100) nach einem der vorhergehenden Ansprüche, ferner umfassend eine Anzeige (190), die insbesondere als Touch-Display ausgebildet ist, wobei mittels der Anzeige (190) die Strahlungsvorrichtung (100) steuerbar ist.

13. Strahlungsvorrichtung (100) nach einem der vorhergehenden Ansprüche, wobei eine innere Oberfläche der Strahlungskammer (150) zumindest teilweise, vorzugsweise im Wesentlichen vollständig, eine freie Oberflächenenergie von maximal 40 mN/m, vorzugsweise 30 mN/m, besonders bevorzugt 25 mN/m aufweist.

14. Strahlungsvorrichtung (100) nach Anspruch 13, wobei die innere Oberfläche der Strahlungskammer (150) zumindest teilweise, vorzugsweise im Wesentlichen vollständig einen Reflexionsgrad von mindestens 80 %, vorzugsweise 90 %, besonders bevorzugt 93 % aufweist.

15. Strahlungsvorrichtung (100) nach einem der Ansprüche 13 oder 14, wobei die innere Oberfläche der Strahlungskammer (150) zumindest teilweise, vorzugsweise im Wesentlichen vollständig aus Polytetrafluorethylen, vorzugsweise aus gesintertem Polytetrafluorethylen ausgebildet ist.

16. Strahlungsvorrichtung (100) nach einer Kombination aus den Ansprüchen 1 und 5, insbesondere in Verbindung mit einem oder mehreren der Ansprüche 2 bis 4 und, 6 15.

17. Verfahren zum Überwachen einer Strahlungsvorrichtung (100) zum Desinfizieren von Zahntechnik, insbesondere eine Strahlungsvorrichtung (100) nach einem der vorhergehenden Ansprüche, umfassend die Schritte des:
- Erfassens der Parameter der zu desinfizierenden Zahntechnik,
- Startens des Betriebs der Strahlungsvorrichtung (100),
- kontinuierlichen Erfassens der Strahlungsdichte in der Strahlungskammer (150),
- Auswertens der erfassten Strahlungsdichte in Abhängigkeit der erfassten Zahntechnikparamter, und
- Ausgebens einer Fehlermeldung bei einer unerwarteten Abweichung der erfassten Strahlungsdichte während der Auswertung.

18. Verfahren nach Anspruch 17, bei dem das Auswerten der erfassten Strahlungsdichte kontinuierlich während des Betriebs der Strahlungsvorrichtung (100) ausgeführt wird.
